# EUROPEAN PATENT SPECIFICATION

(11) **EP 1 621 912 B1**
(45) Date of publication and mention of the grant of the patent: **23.05.2007**
(21) Application number: 05251443.7
(22) Date of filing: 10.03.2005
(51) Int. Cl.: G02B 21/32

(54) **Device for injecting substance into cell and method for injecting substance into cell**
Gerät und Methoden zur Injektion von Substanzen in Zellen
Appareil et méthodes pour l'injection de substances dans cellules

(30) Priority: 27.07.2004 JP 2004219232
(43) Date of publication of application: 01.02.2006
(73) Proprietor: FUJITSU LIMITED, Kawasaki-shi, Kanagawa 211-8588 (JP)
(72) Inventor: Youoku, Sachihiro, c/o Fujisu Limited, Kawasaki-shi Kanagawa 211-8588 (JP); Sasaki, Jun, c/o Fujisu Limited, Kawasaki-shi Kanagawa 211-8588 (JP); Tamamushi, Kazuo, c/o Fujisu Limited, Kawasaki-shi Kanagawa 211-8588 (JP); Ito, Akio, c/o Fujisu Limited, Kawasaki-shi Kanagawa 211-8588 (JP)
(74) Representative: Fenlon, Christine Lesley

(56) References cited:
- EP-A- 1 479 759
- WO-A-00/34436
- US-A- 4 907 158
- US-A- 5 114 854
- US-A- 5 225 750
- US-A1- 2002 116 732
- PATENT ABSTRACTS OF JAPAN vol. 2003, no. 12, 5 December 2003 (2003-12-05) & JP 2004 166653 A (FUJITSU LTD), 17 June 2004 (2004-06-17)

## Description

### BACKGROUND OF THE INVENTION

### 1) Field of the Invention

The present invention relates to an injection device that captures a cell transported through a flow path and injects a substance into the cell captured with a minute needle.

### 2) Description of the Related Art

Recently, in the field of life science, specifically in the fields of the regenerative medicine and the genome-based drug discovery, modification of property of a cell has been practiced by injecting a gene and a drug solution into a cell. Such a technology enables to elucidate a function of a gene, and also enables a tailor-made medicine, which provides a care suitable for a genetic characteristic of an each person.

There are proposed various methods of injecting gene into a cell, such as an electric method (electroporation), a chemical method (lypofection), a biological method (vector method), a mechanical method (microinjection), and an optical method (laser injection).

However, these methods have respective disadvantages. The electric method tends to deeply damage a cell. The chemical method has low introduction efficiency. In the biological method, kinds of material to be introduced are limited. Therefore, the mechanical method (namely, injection method) draws attention as the most safety and highly efficient method.

The injection method has an extremely high introduction success rate, which is close to 100%, and has an advantage such that a combination of a cell with an introduction substance is not limited, unlike the chemical method and the biological method. However, the injection method also has a low throughput. In the injection method, even a skilled operator can work on several hundreds of cells with the substance injected per hour at a maximum.

Japanese Patent Application Laid-Open No. 2004-166653 discloses an injection device, in order to compensate for the disadvantage. The injection device captures a cell transported through a flow path and injects a substance into the cell captured with a minute needle. In this injection device, a substance is injected into cells on a conveyor system so as to improve the throughput.

However, in the above conventional technology, a single observation device is used to recognize a cell transported, a cell captured, and acquire position information of the cell captured. Therefore, a problem is that a substance cannot be injected into a cell efficiently.

More specifically, when a cell is transported in a flow path, if the flow path is observed at a low magnification so as to detect the cell, a low magnification decreases a detection resolution of a position of the cell captured, and a success rate of insertion of a minute needle into the cell. Conversely, if the flow path is observed at a high magnification so as to accurate detection of a position of the cell captured, a high magnification narrows a field of view for observing the cell transported through the flow path. Therefore, the cell cannot be detected, and captured.

Thus, when a single observation device is used to recognize a cell transported, a cell captured, and acquire position information of the cell captured, a substance can not be injected into a cell efficiently.

### SUMMARY OF THE INVENTION

Accordingly, it is desirable to solve at least the above problems in the conventional technology.

According to an embodiment of one aspect of the present invention, a device for injecting a substance into a cell includes a flow path through which a cell is transported, a transported-cell detecting unit that detects the cell transported through the flow path, a cell capturing unit that captures the cell detected by the transported-cell detecting unit, a captured-cell detecting unit that detects the cell captured by the cell capturing unit and determines a position of the cell captured, and a substance injecting unit that injects a substance into the cell captured by the cell capturing unit based on the position determined.

According to an embodiment of another aspect of the present invention, a method for injecting a substance into a cell includes first detecting a cell transported through a flow path, capturing the cell detected in the first detecting, second detecting the cell captured in the capturing, determining a position of the cell detected in the second detecting; and injecting a substance into the cell of which the position is determined in the determining.

### BRIEF DESCRIPTION OF THE DRAWINGS

Fig. 1 is a schematic of an injection device according to an embodiment of the present invention;
Fig. 2 is an enlarged view of a central portion of a flow path shown in Fig. 1;
Fig. 3 is an example of a transported-cell detected image;
Fig. 4 is an example of a captured-cell detected image;
Fig. 5 is an example of an optical system; and
Fig. 6 is a flowchart of a process for generating a substance-introduced cell according to the embodiment.

### DETAILED DESCRIPTION

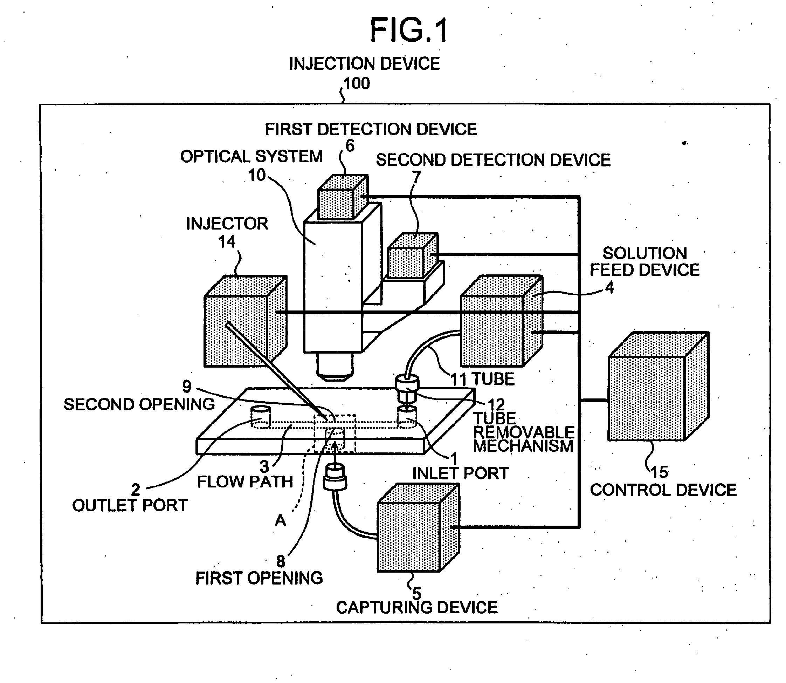

Exemplary embodiments of the present invention will be explained in detail below with reference to the accompanying drawings. First, an overview and characteristics of an embodiment of the present invention will be explained. Fig. 1 is a schematic of an injection device 100 according to an embodiment of the present invention. The injection device 100 captures a cell transported through a flow path and injects a substance into the cell captured with a minute needle.

The characteristics of an embodiment of the present invention is in that the injection device 100 includes a first detection device 6, a capturing device 5, a second detection device 7, and an injector 14. The first detection device 6 detects a cell transported through a flow path, and the capturing device 5 captures the cell based on the result of detection by the first detection device 6. The second detection device 7 detects the cell captured by the capturing device 5 and detects a position of the cell captured, and the injector 14 injects a substance into the cell captured by the capturing device 5 based on the result of detection by the second detection device 7. Consequently, the injection device 100 injects a substance into a cell efficiently.

More specifically, in the injection device 100, the first detection device 6 detects the cell transported through the flow path, and the second detection device 7 detects the cell captured by the capturing device 5 and the position of the cell captured. The first detection device 6 and the second detection device 7 perform the detections independently. Therefore, it is possible to ensure the field of view suitable for recognizing a cell transported while ensuring the field of view suitable for recognizing a cell captured and acquiring position information of the cell captured. Consequently, a capturing-timing detection precision and an introduction success rate improve.

In the conventional technology, an introduction success rate and a capturing-timing detection precision are not high enough, since a single observation device is used to recognize the cell transported, the cell captured, and to acquire position information of the cell captured. On the other hand, in the present invention, the first detection device 6 and the second detection device 7 independently detect a cell transported through the flow path, a cell captured by the capturing device 5, and a position of the cell captured. Therefore, it is possible to respectively ensure the observation fields of view suitable for recognizing a cell transported, a cell captured, and for acquiring position information of the cell captured. Consequently, a capturing-timing detection precision and an introduction success rate improve, and a substance can be efficiently injected into a cell. Furthermore, since the detection of a cell transported and the detection of a cell captured are performed independently from each other, it is possible to confirm the next target cell, which is a cell into which a substance is injected next, while injecting a substance into a cell.

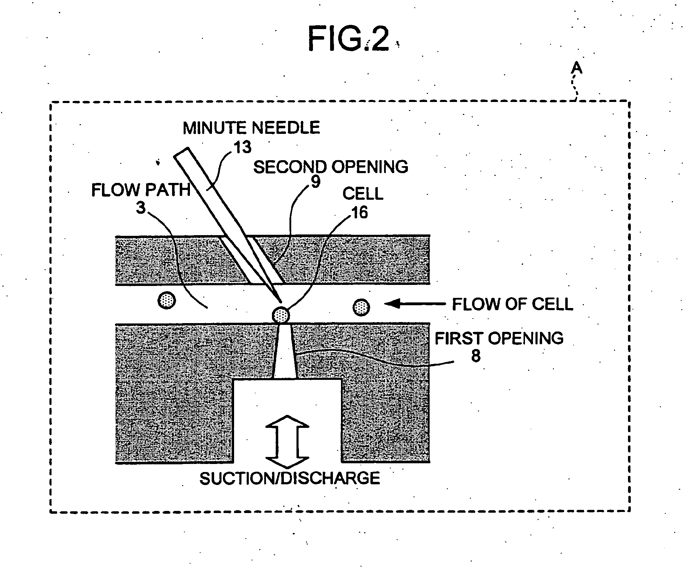

Next, a configuration of the injection device 100 will be explained. Fig. 2 is an enlarged view of a central portion of a flow path (i.e., portion A) in the injection device 100. As shown in Fig. 1, the injection device 100 includes a solution feed device 4, the capturing device 5, the first detection device 6, the second detection device 7, the injector 14, and a control device 15.

The solution feed device 4 controls a solution feed amount and a solution feed speed of cell suspension, which is solution that contains cells transported into a flow path 3. The solution feed device 4 is realized by a known liquid chromatograph pump and so on.

More specifically, the solution feed device 4 is connected to an inlet port 1 of the flow path 3 via a tube 11 and a removable mechanism 12, which is fixed to the end of the tube 11, and controls a discharge amount to control the solution feed amount and the solution feed speed of the cell suspension. Such a solution feed speed is preferably about 300 µm/sec on an average.

Although the solution feed device 4 is connected to the inlet port 1 of the flow path 3 to discharge the solution, the solution feed device 4 may be connected to an outlet port 2 of the flow path 3 and control a suction amount to transport the cell suspension.

The first detection device 6 is used to observe a cell and detect a cell transported through the flow path 3. Specifically, the first detection device 6 detects, through an optical system 10, whether a cell 16 is transported in the flow path 3, and transmits a transported-cell detected signal to the control device 15 when the cell 16 is detected in the flow path 3.

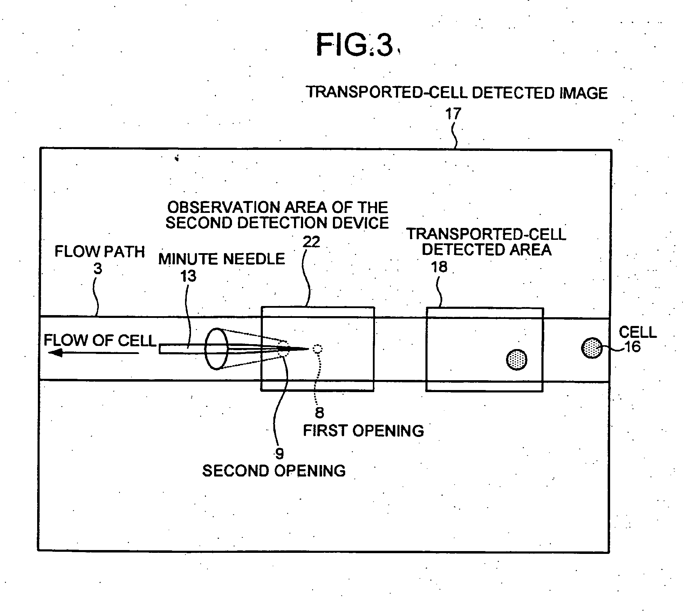

More specifically, the first detection device 6 takes a transported-cell detected image 17 (see Fig. 3) through the optical system 10 by using a Charge-Coupled Device camera (hereinafter, "CCD camera") at a predetermined time interval. The first detection device 6 specifies a transported-cell detected area 18 in the transported-cell detected image 17. The first detection device 6 compares the respective transported-cell detected areas 18 of the respective transported-cell detected images 17 that is sequentially acquired, with one another, and monitors change of brightness in the transported-cell detected area 18 so as to detect the cell 16. Although it is desirable that the first detection device 6 take the transported-cell detected image 17 at a rate of 30 frames/sec or more, the embodiment of the present invention is not restricted by the description.

In order to prevent erroneous detection of impurities other than cells, when the cell 16 is to be detected, a desired condition is applied to the size of an area where the brightness changes in the transported-cell detected area 18.

The second detection device 7 detects a cell captured by the capturing device 5 and detects position of the cell captured. Specifically, the second detection device 7 detects, through the optical system 10, whether the cell 16 is captured in the flow path 3, and transmits, to the control device 15, a captured-cell detected signal and position information of the call captured when the cell 16 captured is detected in the flow path 3.

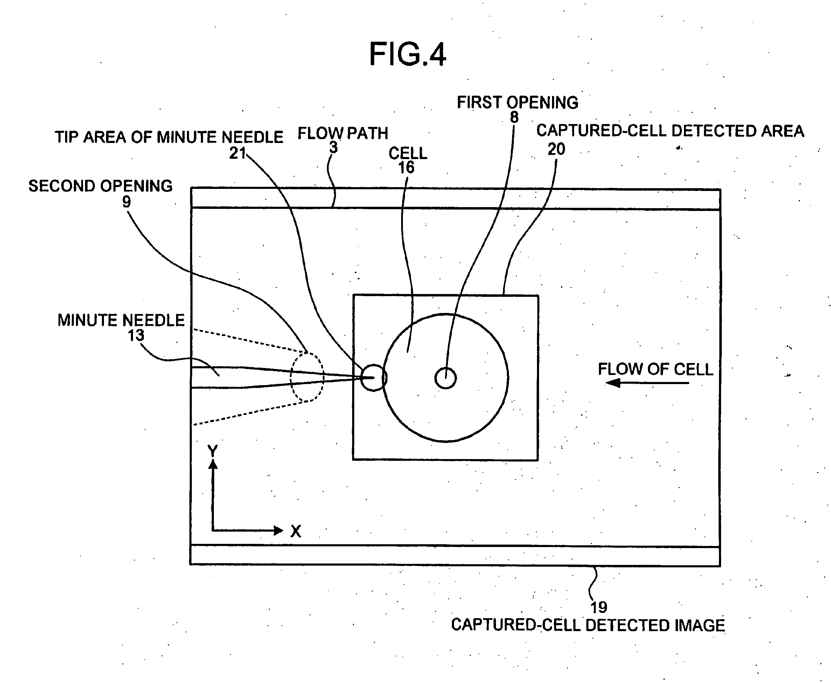

More specifically, the second detection device 7 takes a captured-cell detected image 19 (see Fig. 4) through the optical system 10 by using a CCD camera at a predetermined time interval. The second detection device 7 specifies a captured-cell detected area 20 in the captured-cell detected image 19. The second detection device 7 compares the respective captured-cell detected areas 20 of the respective captured-cell detected images 19 that are acquired sequentially, with one another, and monitor change in brightness in the captured-cell detected area 20 so as to detect the cell 16 captured. Although it is desirable that the second detection device 7 take the captured-cell detected images 19 at a rate of 30 frames/sec or more, the embodiment of the present invention is not restricted by the description.

In order to prevent erroneous detection of impurities other than cells, when the cell 16 is to be detected, a desired condition is applied to the size of an area where the brightness changes in the captured-cell detected area 20.

Furthermore, by performing image processing so as to enhance an outline of the cell 16 in the captured-cell detected image 19, the second detection device 7 acquires a shape of the cell 16 and coordinates of the position where the cell 16 is captured. Since a flow-type cell is generally spherical, and the central portion of the flow-type cell is transparent, a pixel position (X1, Y1) at the center of the cell 16 can easily be detected on an image by enhancing the outline of the cell 16 through the image processing. On the image, the pixel position (X1, Y1) at the center of the cell 16 is compared with a position (X2, Y2) of a minute needle 13 so as to decide the amount of movement of the minute needle 13.

The second detection device 7 extracts a tip area 21 of the minute needle in the captured-cell detected image 19, and measures the shape of the tip area 21 and the amount of an introduction substance that is discharged from the tip area 21. For example, in order to check a state of how the introduction substance is discharged, a stain or a drug solution that contains a fluorescent sample is used for the introduction substance. When the stain is used, the discharge is confirmed, through bright-field observation, by checking a state of how the color around the tip area of the minute needle changes upon its discharge. When the fluorescent sample is used, the discharge is confirmed, through fluorescence observation, by checking whether there is fluorescence around the tip area of the minute needle upon its discharge.

By detecting the shape of the minute needle 13 of the injector 14 and a state of how the introduction substance is discharged from the tip of the minute needle 13 in the above manner, it is possible to detect a breakage of the minute needle 13 and a clogging of the introduction substance at the tip area 21 of the minute needle 13.

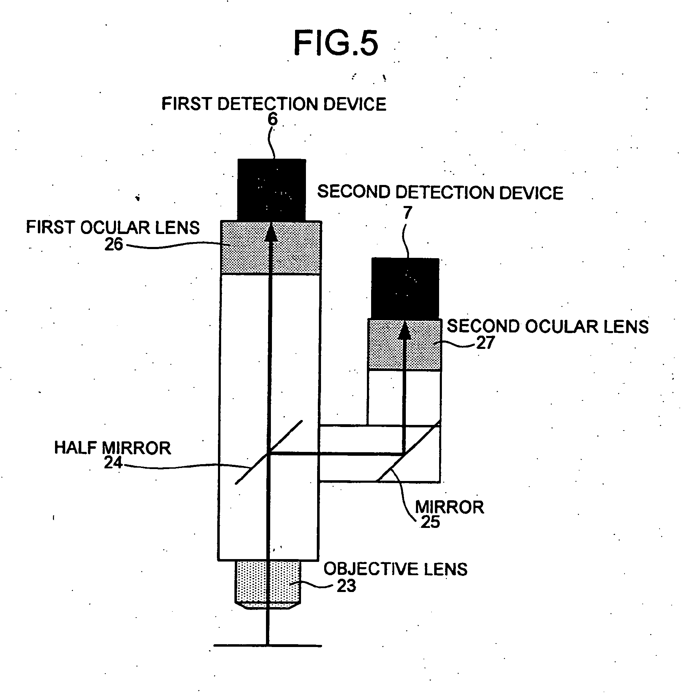

An example of an optical system will be explained below. In the optical system shown in Fig. 5, one optical system is branched to form the first detection device 6 and the second detection device 7. Light enters through an objective lens 23, and is split into light that passes through a half mirror 24 and light that is refracted by 90 degrees by the half mirror 24. The light that passes through the half mirror 24 is converged by a first ocular lens 26, and enters into the first detection device 6. On the other hand, the light that is refracted by the half mirror 24 is further refracted by 90 degrees by a mirror 25, is converged by a second ocular lens 27, and enters into the second detection device 7.

By changing each magnification of the first ocular lens and the second ocular lens, each area of observation fields of view of the first detection device 6 and the second detection device 7 can be arbitrarily changed. A magnification ratio between the first ocular lens and the second ocular lens is desirably 4:1 or higher.

As explained above, the first detection device 6 and the second detection device 7 recognize on the respective observation images that are taken in a different magnification through the same optical system 10, and detect a cell transported through the flow path, a cell captured by the capturing device 5, and position information of the cell captured, respectively. It is thereby possible to acquire observation images in which the respective optical axes of the first detection device 6 and the second detection device 7 are coincident with each other, and to obtain the result of accurate detection. Moreover, it is possible to configure these detection devices at low cost.

The capturing device 5 captures a cell transported through the flow path when receiving a capture-start signal from the control device 15. Specifically, the capturing device 5 is connected to a first opening 8 in the flow path 3, and controls a suction amount and a discharge amount of the cell suspension so as to capture the cell 16 transported into the flow path at the first opening 8. A diameter of the first opening 8 is not more than a diameter of a cell. For example, if the diameter of the cell 16 is 15 µm, the diameter of the first opening 8 is preferably not more than 5 µm so as to capture a cell with high probability.

When the first detection device 6 detects a cell transported through the flow path in the above manner, the process of capturing the cell starts. Therefore, a cell is automatically captured, and by checking whether a cell is present at a particular position of the flow path, the timing of capturing a cell is controlled.

The injector 14 injects a substance into the cell captured by the capturing device 5 when receiving an injection-start signal from the control device 15. Specifically, the injector 14 controls the minute needle 13, which is used to inject an introduction substance, and inserts the minute needle 13 into the flow path 3 through a second opening 9 of the flow path so as to inject an introduction substance into the cell 16 captured at the first opening 8. Although a diameter of the second opening 9, through which the minute needle 13 is inserted, is preferably about 20 µm in an embodiment of the present invention, the embodiment of the present invention is not restricted by the description.

When the second detection device 7 detects the cell captured by the capturing device 5, a process of injecting a substance into a cell starts. Therefore, an introduction substance is injected into a cell automatically.

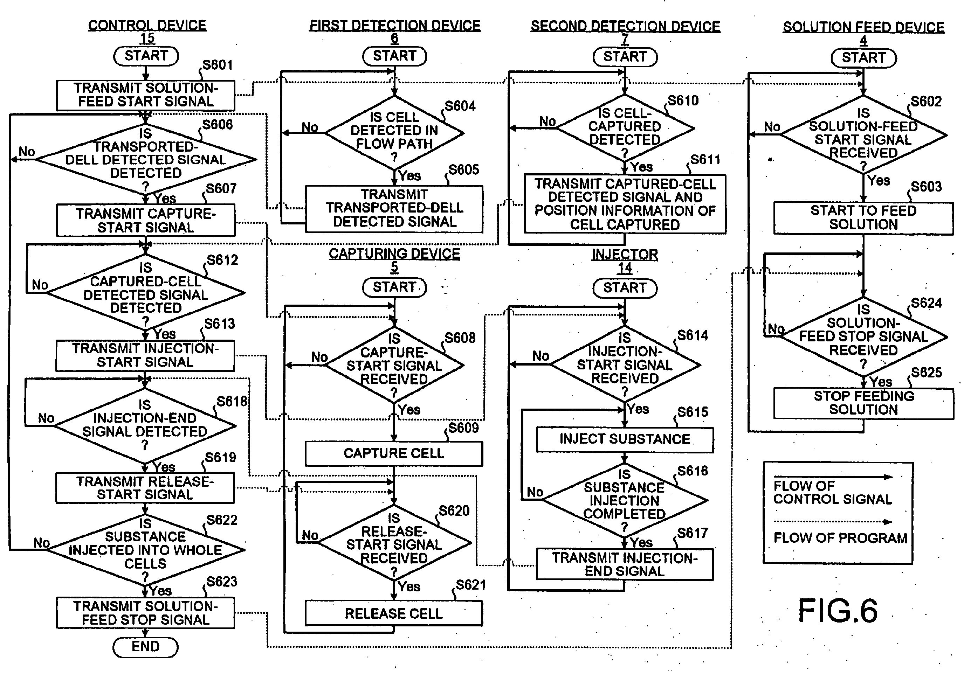

Procedures of various processes of the injection device according to the embodiment will be explained below. Fig. 6 is a flowchart of a process for generating of a substance-introduced cell according to the embodiment. The process of generating a substance-introduced-cell starts after the solution feed device 4 or the inlet port 1 of the flow path is filled with the cell suspension. If an adherent cell is used, a trypsin treatment or the like is performed so that cells are peeled off from a carrier, and dispersed and floating in a liquid.

After filled with the cell suspension, the control device 15 transmits a solution-feed start signal to the solution feed device 4 at step S601. If it is determined, at step S602, that the solution feed device 4 receives the solution-feed start signal, the solution feed device 4 then starts to feed the cell suspension to the flow path 3 at step S603.

The first detection device 6 recursively performs detection operation of the cell 16 transported through the flow path 3. If it is determined, at step S604, that the first detection device 6 detects the cell 16 in the flow path 3, the first detection device 6 transmits the transported-cell detected signal to the control device 15 at step S605.

Then, if it is determined, at step S606, that the control device 15 receives the transported-cell detected signal from the first detection device 6, the control device 15 transmits the capture-start signal to the capturing device 5 at step S607. If it is determined, at step S608, that the capturing device 5 receives the capture-start signal from the control device 15, the capturing device 5 generates negative pressure on the first opening 8 of the flow path 3 connected thereto through the tube 11, and captures the cell 16 at the first opening 8 at step S609.

The second detection device 7 recursively performs detection operation of the cell 16 captured at the first opening 8 of the flow path 3. If it is determined, at step S610, that the second detection device 7 detects the cell captured at the first opening, the second detection device 7 transmits the captured-cell detected signal and the position information of the cell captured to the control device 15 at step S611.

Then, it is determined, at step S612, that the control device 15 receives the captured-cell detected signal from the second detection device 7, the control device 15 calculates the amount of movement of the minute needle 13 in the injector 14 so as to insert the minute needle 13 into the cell captured, based on the position information of the cell captured obtained by the second detection device 7, and transmits the injection-start signal and information about the amount of movement of the minute needle 13, to the injector 14 at step S613.

When it is determined, at step S614, that the injector 14 receives the injection-start signal from the control device 15, the injector 14 moves the minute needle 13 according to the amount of movement thereof received together with the injection-start signal, inserts the minute needle 13 into the cell captured, and injects the introduction substance at step S615.

Thereafter, when it is determined, at step S616, that the injector 14 completes the injection of the introduction substance, the injector 14 transmits an injection-end signal to the control device 15 at step S617. When it is determined, at step S618, that the control device 15 receives the injection-end signal from the injector 14, the control device 15 transmits a release-start signal to the capturing device 5 at step S619.

Then, if it is determined, at step S620, that the capturing device 5 receives the release-start signal from the control device 15, the capturing device 5 generates positive pressure on the first opening 8 of the flow path 3 connected thereto through the tube 11, and releases the cell 16 captured at the first opening 8 at step S621. The cell 16 is released, and then transported by the solution feed device 4 to the outlet port 2 of the flow path 3.

If it is determined, at step S622, that injecting the substance into the whole cells filled is completed, the control device 15 transmits a solution-feed stop signal to the solution feed device 4 at step S623. If the first detection device 6 does not detect the cell in the flow path 3 for a predetermined time or if injecting substance into a preset number of cells is completed, it is determined that injecting the substance into the whole cells is completed.

Finally, when it is determined, at step S624, that the solution feed device 4 receives the solution-feed stop signal from the control device 15, the solution feed device 4 stops feeding the solution at step S625, and ends the process of generating a substance-introduced cell.

As explained above, in the injection device 100 according to the embodiment, the first detection device 6 detects a cell transported through the flow path, the second detection device 7 detects a cell captured by the capturing device 5 and position information of the cell captured, and these detections are performed independently from each other. Therefore, it is possible to ensure the observation field of view suitable for recognizing the cell transported while ensuring the observation field of view suitable for recognizing the cell captured and suitable for acquiring the position information of the cell captured. Consequently, a capturing-timing detection precision and an introduction success rate improve, and it is possible to efficiently inject a substance into a cell. Furthermore, since detection of a cell transported and detection of a cell captured are performed independently from each other, it is possible to confirm a next target cell, which is a cell into which a substance is injected next, while injecting the substance into a cell.

When using the observation system that uses the first detection device and the second detection device according to the embodiment, the flow path is preferably formed with a transparent material to carry out transparent observation. However, the embodiment of the present invention is not restricted by the description. For example, if the flow path is illuminated from the above and observed, the material of the flow path does not need to be transparent.

Furthermore, although, in the explanation of the embodiment, control signals are transmitted and received through the control device 15, embodiments of the present invention are not limited thereto. The control signal may be transmitted or received between the devices.

Among the processes explained in the embodiment, the whole or a part of the processes has been explained assuming that it is automatically performed, but it can also be performed manually. Alternatively, the whole or a part of the processes has been explained assuming that it is manually performed, but it can also be performed automatically using the known method. In addition to these, the information that includes the process procedures, the control procedures, the specific names, and the various data and parameters as shown in the specification and the figures can arbitrarily be changed unless otherwise specified.

The components of the devices as shown in the figures are only conceptual functions, and therefore, they are not always configured physically as shown in the figures. In other words, a specific arrangement obtained by separation or integration of the devices is not limited by the arrangements in the figures. Therefore, the whole or a part of the devices can be functionally or physically separated or integrated as arbitrary units according to various loads or their statuses.

The injection device according to an embodiment of the present invention is useful for an injection device that captures a cell transported through a flow path and injects a substance into the cell captured with a minute needle. Particularly, an embodiment of the present invention is suitable for an injection device used for medical application such as regenerative medicine and genome-based drug discovery and so on.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

## Claims

1. A device for injecting a substance into a cell, comprising:
a flow path through which a cell is transported;
a transported-cell detecting unit that detects the cell transported through the flow path;
a cell capturing unit that captures the cell detected by the transported-cell detecting unit;
a captured-cell detecting unit that detects the cell captured by the cell capturing unit and determines a position of the cell captured; and
a substance injecting unit that injects a substance into the cell captured by the cell capturing unit based on the position determined.

2. The device according to claim 1, wherein when the transported-cell detecting unit detects the cell transported through the flow path, the cell capturing unit starts to capture the cell transported through the flow path.

3. The device according to claim 1 or 2, wherein when the captured-cell detecting unit detects the cell captured by the cell capturing unit, the substance injecting unit starts to inject the substance into the cell captured by the cell capturing unit.

4. The device according to claim 1, 2 or 3, further comprising:
a first image acquiring unit that that acquires a first observation image of an area in the flow path at a first magnification through an optical system; and
a second image acquiring unit that that acquires a second observation image of the cell captured by the cell capturing unit at a second magnification through the optical system, wherein the first magnification is different from the second magnification, and
the transported-cell detecting unit detects the cell based on the first observation image, and
the captured-cell detecting unit detects the cell and determines the position based on the second observation image.

5. The device according to any preceding claim, wherein
the substance injecting unit includes a needle, and
the captured-cell detecting unit further detects a shape of the needle and a state of how an introduction substance is discharged from a tip of the minute needle.

6. The device according to any preceding claim, wherein
the transported-cell detecting unit detects a next target cell transported through the flow path while the substance injecting unit injects a substance into the cell captured by the cell capturing unit based on the position detected, the next target cell being a cell into which the substance injecting unit injects a substance next.

7. A method for injecting a substance into a cell, comprising:
first detecting a cell transported through a flow path;
capturing the cell detected in the first detecting;
second detecting the cell captured in the capturing;
determining a position of the cell detected in the second detecting; and
injecting a substance into the cell of which the position is determined in the determining.

8. The method according to claim 7, further comprising third detecting a next target cell transported through the flow path while the substance is being injected into the cell in the injecting, the next target cell being a cell into which a substance is to be injected next.

## Patentansprüche

1. Vorrichtung zum Injizieren einer Substanz in eine Zelle, die umfasst:
einen Fließweg, durch den eine Zelle transportiert wird;
eine Detektionseinheit der transportierten Zelle, die die Zelle detektiert, die durch den Fließweg transportiert wird;
eine Zellenfangeinheit, die die Zelle fängt, die durch die Detektionseinheit der transportierten Zelle detektiert wurde;
eine Detektionseinheit der gefangenen Zelle, die die Zelle detektiert, die durch die Zellenfangeinheit gefangen wurde, und eine Position der gefangenen Zelle bestimmt; und
eine Substanzinjektionseinheit, die eine Substanz in die Zelle, die durch die Zellenfangeinheit gefangen wurde, auf der Basis der bestimmten Position injiziert.

2. Vorrichtung nach Anspruch 1, bei der dann, wenn die Detektionseinheit der transportierten Zelle die Zelle detektiert, die durch den Fließweg transportiert wird, die Zellenfangeinheit beginnt, die Zelle zu fangen, die durch den Fließweg transportiert wird.

3. Vorrichtung nach Anspruch 1 oder 2, bei der dann, wenn die Detektionseinheit der gefangenen Zelle die Zelle detektiert, die durch die Zellenfangeinheit gefangen wurde, die Substanzinjektionseinheit beginnt, die Substanz in die Zelle zu injizieren, die durch die Zellenfangeinheit gefangen wurde.

4. Vorrichtung nach Anspruch 1, 2 oder 3, die ferner umfasst:
eine erste Bilderfassungseinheit, die ein erstes Beobachtungsbild eines Bereiches in dem Fließweg mit einer ersten Vergrößerung durch ein optisches System erfasst; und
eine zweite Bilderfassungseinheit, die ein zweites Beobachtungsbild der durch die Zellenfangeinheit gefangenen Zelle mit einer zweiten Vergrößerung durch das optische System erfasst, wobei sich die erste Vergrößerung von der zweiten Vergrößerung unterscheidet, und
die Detektionseinheit der transportierten Zelle auf der Basis des ersten Beobachtungsbildes die Zelle detektiert und
die Detektionseinheit der gefangenen Zelle auf der Basis des zweiten Beobachtungsbildes die Zelle detektiert und die Position bestimmt.

5. Vorrichtung nach irgendeinem vorhergehenden Anspruch, bei der
die Substanzinjektionseinheit eine Nadel enthält und
die Detektionseinheit der gefangenen Zelle ferner eine Form der Nadel und einen Zustand dessen detektiert, wie eine Einführungssubstanz von einer Spitze der winzigen Nadel entladen wird.

6. Vorrichtung nach irgendeinem vorhergehenden Anspruch, bei der
die Detektionseinheit der transportierten Zelle eine nächste Zielzelle detektiert, die durch den Fließweg transportiert wird, während die Substanzinjektionseinheit eine Substanz in die durch die Zellenfangeinheit gefangene Zelle auf der Basis der detektierten Position injiziert, wobei die nächste Zelle eine Zelle ist, in die die Substanzinjektionseinheit als Nächstes eine Substanz injiziert.

7. Verfahren zum Injizieren einer Substanz in eine Zelle, mit den Schritten:
erstes Detektieren einer Zelle, die durch einen Fließweg transportiert wird;
Fangen der Zelle, die beim ersten Detektieren detektiert wurde;
zweites Detektieren der Zelle, die beim Fangen gefangen wurde;
Bestimmen einer Position der Zelle, die beim zweiten Detektieren detektiert wurde; und
Injizieren einer Substanz in die Zelle, deren Position beim Bestimmen bestimmt wurde.

8. Verfahren nach Anspruch 7, ferner mit dem dritten Detektieren einer nächsten Zielzelle, die durch den Fließweg transportiert wird, während beim Injizieren die Substanz in die Zelle injiziert wird, wobei die nächste Zielzelle eine Zelle ist, in die als Nächstes eine Substanz zu injizieren ist.

## Revendications

1. Dispositif destiné à injecter une substance dans une cellule, comprenant:
un trajet d'écoulement au travers duquel une cellule est transportée ;
une unité de détection de cellules transportées qui détecte la cellule transportée au travers du trajet d'écoulement ;
une unité de capture de cellules qui capture la cellule détectée par l'unité de détection de cellules transportées ;
une unité de détection de cellules capturées qui détecte la cellule capturée par l'unité de capture de cellules et détermine une position de la cellule capturée ; et
une unité d'injection de substance qui injecte une substance dans la cellule capturée par l'unité de capture de cellules sur la base de la position déterminée.

2. Dispositif selon la revendication 1, dans lequel, lorsque l'unité de détection de cellules transportées détecte la cellule transportée au travers du trajet d'écoulement, l'unité de capture de cellules commence à capturer la cellule transportée au travers du trajet d'écoulement.

3. Dispositif selon la revendication 1 ou 2, dans lequel, lorsque l'unité de détection de cellules capturées détecte la cellule capturée par l'unité de capture de cellule, l'unité d'injection de substance commence à injecter la substance dans la cellule capturée par l'unité de capture de cellules.

4. Dispositif selon la revendication 1, 2 ou 3, comprenant en outre :
une première unité d'acquisition d'image qui acquiert une première image d'observation d'une zone dans le trajet d'écoulement à un premier niveau de grossissement au travers d'un système optique ; et
une seconde unité d'acquisition d'image qui acquiert une seconde image d'observation de la cellule capturée par l'unité de capture de cellules à un second niveau de grossissement au travers du système optique, dans lequel le premier grossissement est différent du second grossissement, et
l'unité de détection de cellules transportées détecte la cellule sur la base de la première image d'observation, et
l'unité de détection de cellules capturées détecte la cellule et détermine la position sur la base de la seconde image d'observation.

5. Dispositif selon l'une quelconque des revendications précédentes, dans lequel
l'unité d'injection de substance comporte une aiguille, et
l'unité de détection de cellules capturées détecte en outre une forme de l'aiguille et un état dans lequel une substance d'introduction est déchargée d'une pointe de la minuscule aiguille.

6. Dispositif selon l'une quelconque des revendications précédentes, dans lequel
l'unité de détection de cellules transportées détecte une cellule cible suivante transportée au travers du trajet d'écoulement, alors que l'unité d'injection de substance injecte une substance dans la cellule capturée par l'unité de capture de cellules sur la base de la position détectée, la cellule cible suivante étant une cellule dans laquelle l'unité d'injection de substance injecte ensuite une substance.

7. Procédé destiné à injecter une substance dans une cellule, comprenant :
une première détection d'une cellule transportée au travers d'un trajet d'écoulement ;
une capture de la cellule détectée au cours de la première détection ;
une deuxième détection de la cellule capturée au cours de la capture ;
une détermination d'une position de la cellule détectée au cours de la deuxième détection ; et
une injection d'une substance dans la cellule dont la position est déterminée au cours de la détermination.

8. Procédé selon la revendication 7, comprenant en outre une troisième détection d'une cellule cible suivante transportée au travers du trajet d'écoulement alors que la substance est injectée dans la cellule au cours de l'injection, la cellule cible suivante étant une cellule dans laquelle une substance doit être injectée ensuite.
